# EUROPÄISCHE PATENTSCHRIFT

(11) **EP 0 946 150 B1**
(45) Veröffentlichungstag und Bekanntmachung des Hinweises auf die Patenterteilung: **06.03.2002**
(21) Anmeldenummer: 97944810.7
(22) Anmeldetag: 29.08.1997
(51) Int. Cl.: A61K 9/20

(54) **ORALE ZUBEREITUNG, ENTHALTEND IN EINER IN WÄSSRIGEM MEDIUM QUELLBAREN MATRIX WENIGSTENS EINEN PHARMAZEUTISCHEN WIRKSTOFF**
ORAL PREPARATION CONTAINING AT LEAST ONE ACTIVE PHARMACEUTICAL SUBSTANCE IN A MATRIX CAPABLE OF SWELLING IN AN AQUEOUS MEDIUM
PREPARATION ORALE RENFERMANT, DANS UNE MATRICE CAPABLE DE GONFLEMMENT EN MILIEU AQUEUX, AU MOINS UNE SUBSTANCE ACTIVE PHARMACEUTIQUE

(30) Priorität: 28.09.1996 DE 19640062
(43) Veröffentlichungstag der Anmeldung: 06.10.1999
(73) Patentinhaber: LTS LOHMANN Therapie-Systeme AG, 56626 Andernach (DE)
(72) Erfinder: CREMER, Karsten, D-53119 Bonn (DE)
(74) Vertreter: Flaccus, Rolf-Dieter, Dr.
(86) Internationale Anmeldenummer: EP9704717
(87) Internationale Veröffentlichungsnummer: WO9813028

(56) Entgegenhaltungen:
- EP-A- 0 795 324
- WO-A-94/02121
- WO-A-94/21236
- VINCENT LENAERTS, ET AL.: "Controlled Release of theophylline from cross-linked amylose tablets" JOURNAL OF CONTROLLED RELEASE, Bd. 15, Nr. 1, 1991, AMSTERDAM NL, Seiten 39-46, XP000200508

## Beschreibung

Die Erfindung betrifft eine orale Zubereitung, enthaltend in einer in wässrigem Medium quellbaren Matrix wenigstens einen pharmazeutischen Wirkstoff, der aus der Matrix retardiert in das wässrige Meedium freigesetzt wird, während die Matrix weitgehend zerfallsresistent bleibt.

Die retardierte Freisetzung von Wirkstoff aus peroralen Darreichungsformen dient der Vermeidung von unerwünschten Plasmaspiegelschwankungen. Zu den Gestaltungsvarianten, mit denen sich eine retardierte Wirkstofffreisetzung erzielen läßt, zählen auch Schichttabletten, insbesondere solche, bei denen sich die Oberfläche der wirkstoffhaltigen Schicht im Verlauf der Freisetzung vergrößert, wodurch die Freisetzungsgeschwindigkeit derart gesteuert werden kann, daß sie weitgehend konstant bleibt. Diese Oberflächenvergrößerung kann je nach dem Design und der Rezeptur der Schichttablette aus der kontinuierlichen Erosion einer oder mehrerer benachbarter Schichten oder aus der Quellung der wirkstoffhaltigen Schicht resultieren. Das erstgenannte Prinzip wurde in den Dokumenten P 43 41 442.7 und P 44 16 926.4 beschrieben, das zweite beispielsweise in der US 5,422,123.

Bei der Untersuchung handelsüblicher Schichttabletten mit quellbarer Matrixschicht wurde als wesentlich erkannt, daß eine zufriedenstellende Oberflächenvergrößerung durch Quellung nur dann zu erreichen ist, wenn eine mechanische Beanspruchung der Tablette weitgehend vermieden wird. Wenn nämlich eine Quellung bei Zutritt von wäßrigem Medium stattfindet, entsteht unter Volumenzunahme ein halbfestes Gel mit vergleichsweise geringer mechanischer Stabilität. Bereits die Durchführung der Zerfallsprüfung nach DAB 10 mit aufgelegten Plexiglasscheiben führt zu einem gravierenden Formverlust der gequollenen Tabletten.

Ursache für den Mangel an mechanischer Stabilität solcher Matrixschichten in gequollenem Zustand ist die Verwendung von quellbaren, eine Quellung hervorrufenden Polymeren. Es handelt sich vorwiegend um stark hydrophile Polymere, die jedoch durch Quervernetzung, durch Ausbildung von kristallinen Bezirken oder durch ihre große Kettenlänge unlöslich oder langsam löslich sind. Beispiele für diese Typen von Polymeren sind Croscarmellose, Polyvinylalkohol mit hohem Hydrolysegrad und hochmolekulare Hydroxypropylmethylcellulose. Diese sehr stark quellbaren Polymere werden aufgrund dieser Eigenschaft auch als Zerfallsbeschleuniger bzw. Sprengmittel in Tablettenrezepturen eingesetzt (Sucker et al., Pharmazeutische Technologie, Thieme Verlag, 1991, S.174 f.).
Die Herstellung von quellbaren Matrizes, die bei ausreichender Quellung im wäßrigen Medium gleichzeitig mechanisch stabil bleiben, ist unter Verwendung der genannten Polymere äußerst problematisch, weil deren quellungsfördernden Eigenschaften von zerfallsfördernden Eigenschaften nicht zu trennen sind.

Aufgabe der vorliegenden Erfindung ist es daher, eine perorale Zubereitung mit einer bei Zutritt von wäßrigem Medium quellbaren Matrix zu schaffen, aus welcher Wirkstoff retardiert in das wäßrige Medium freigesetzt wird, und die im gequollenen Zustand bis zum weitgehenden Ende der Freisetzung mechanisch stabil und zerfallsresistent ist.

Die Aufgabe wird bei einer Zubereitung der im Oberbegriff von Anspruch 1 bezeichneten Art erfindungsgemäß mit den kennzeichnenden Merkmalen des Hauptanspruchs gelöst.

Quervernetzte Amylose quillt in Wasser, unterscheidet sich aber von den üblichen quellbaren Polymeren durch eine überraschend hohe mechanische Festigkeit in gequollenem Zustand. Auf diese Weise ist es erstmalig möglich, deutlich verbesserte, zerfallsresistente Schichttabletten zu schaffen, bei denen die Oberflächenvergrößerung der quellenden Matrixschicht auch dann noch wirksam ist, wenn mechanische Beanspruchungen auftreten, wie z.B. bei der Zerfallsprüfung oder nach der Applikation im Gastrointestinaltrakt.

Amylose ist Bestandteil von natürlichen Stärken, die mit einem Gehalt von etwa 15 bis 30 % vorwiegend im Innern von Stärkekörnern enthalten ist, und besteht aus unverzweigten Ketten mit D-Glucopyranosebausteinen, die α-1,4-glycosidisch miteinander verknüpft sind. Durch Reaktion mit Agentien wie z.B. Epichlorhydrin oder 2,3-Dibrompropanol läßt sich Amylose wirksam quervernetzen, wofür ein Mengenanteil von 0,1 bis 10 % Quervernetzer, bezogen auf die Amylose, erforderlich ist.

Aus der US-Patentschrift 5,456,921 ist bekannt, daß quervernetzte Amylose, insbesondere solche mit relativ niedrigem Vernetzungsgrad, sich insbesondere durch eine starke Neigung zur Ausbildung von Wasserstoffbrückenbindungen zwischen den Molekülketten auszeichnet, die wiederum zu großen Kohäsionskräften führt, welche die mechanische Stabilität einer Zubereitung bzw. einer Tablette auch in gequollenem Zustand aufrechterhalten kann.In dieser Patentschrift werden auch verschiedene homogene, d.h. einschichtige Tabletten mit quervernetzter Amylose beschrieben, die ihren Wirkstoff, z.B. Theophyllin, gleichmäßig retardiert freisetzen. Dabei werden für die Steuerung der Freisetzungsgeschwindigkeit unterschiedliche Arten von Mechanismen diskutiert, wobei die favorisierte Art auf einer Steuerung der Kinetik des Wasserzutritts durch intermolekulare Wasserstoffbrückenbindungen-beruht und dieser die größte Bedeutung für die Freisetzungsgeschwindigkeit beigemessen wird.

Überraschenderweise wurde nun mit der Erfindung erstmals erreicht, daß Schichttabletten mit wenigstens einer quellbaren Matrixschicht, wobei deren Freisetzungskinetik über den Oberflächenzuwachs der Matrixschicht durch Quellung gesteuert wird, gegenüber dem Stand der Technik bei einem Gehalt an Amylose ein deutlich erhöhtes Maß an mechanischer Festigkeit besitzen, wodurch eine zuverlässige Steuerung der Freisetzungskinetik auch bei mechanischer Beanspruchung der Tablette gewährleistet ist,weil deren Oberflächenzuwachs trotz dieser Beanspruchung stattfindet, ohne daß sie bei der Freisetzung zerfallen.

Der Begriff Schichttablette im Sinne dieser Erfindung bezieht sich auf eine Tablette aus mindestens zwei jeweils durch Komprimieren von Pulver oder Granulat hergestellten, aufeinander haftenden Schichten. Eine Schichttablette kann zusätzlich weitere Merkmale besitzen, wie etwa eine Umhüllung aus einer zuckerhaltigen oder polymerhaltigen Zusammensetzung oder einen durch Komprimieren von Pulver oder Granulat erzeugten Mantel. In diesen Fällen wäre die Schichttablette auch als Dragee, Filmtablette oder Manteltablette zu bezeichnen.

Die im Kennzeichnungsteil von Anspruch 1 definierte Schichttablette besitzt eine wirkstoffhaltige Matrixschicht, wobei dieser Begriff als übergeordnete Bezeichnung für alle Schichtzusammensetzungen zu verstehen ist, in denen Wirkstoff gleichmäßig verteilt bzw. eingebettet ist. Eine erfindungsgemäße Schichttablette kann auch mehr als eine wirkstoffhaltige Matrixschicht aufweisen, etwa wenn mehr als ein Wirkstoff retardiert, oder verschiedene Wirkstoffanteile mit unterschiedlicher Geschwindigkeit aus unterschiedlichen Schichten freigesetzt werden sollen.

Dementsprechend kann eine erfindungsgemäße Schichttablette einen oder mehrere Wirkstoffe enthalten.

Die wirkstoffhaltige Matrixschicht ist bei Zutritt von wäßrigem Medium quellbar, d.h. bei Kontakt mit Wasser, physiologischem oder künstlichem Magen- oder Darmsaft nimmt die Matrixschicht unter Volumenvergrößerung Wasser und ggf. im Wasser gelöste Bestandteile auf, wobei ein großer Teil des Quellungswassers typischerweise zwischen Polymerketten eingelagert wird, die in einer Matrixzubereitung enthalten sind.

Eine weitgehende Zerfallsresistenz einer Matrixschicht während des Freisetzungsprozesses bedeutet im Sinne der Erfindung, daß der Zerfall bei der Zerfallsprüfung nach DAB 10 nicht abgeschlossen ist, solange nicht zumindest der größere Teil der in der Matrixschicht enthaltenen Wirkstoffdosis freigesetzt wurde. Eine retardierte Wirkstofffreisetzung geschieht in der Regel mindestens über mehrere Stunden, wobei alle Arten von Freisetzungsprofilen, also lineare und nichtlineare, aber auch komplexe Profile mit einer Initial- und einer Erhaltungsdosis usw. eingeschlossen sind.

Die erfindungsgemäße Schichttablette gemäß Hauptanspruch ist dadurch von bekannten Schichttabletten vom Stand der Technik unterschieden, daß sie eine quellbare Matrixschicht mit einem Gehalt an quervernetzten Amylosen aufweist. Bevorzugte Gehalte an quervernetzter Amylose in der Matrixschicht liegen zwischen 30 und 99,5 %. Während sehr niedrig zu dosierende Wirkstoffe einen hohen Gehalt an quervernetzter Amylose erlauben, verlangen größere Wirkstoffdosen im Hinblick auf eine maximal applizierbare Tablettengröße eine mehr oder weniger deutliche Reduktion des Hilfsstoffgehalts. Um die oben beschriebenen, positiven Effekte der quervernetzten Amylose zum Tragen kommen zu lassen, wird man jedoch in der Regel einen Gehalt von mindestens 20 % einsetzen müssen. Noch niedrigere Einsatzmengen sind zwar weniger bevorzugt, entsprechen jedoch ebenfalls der Erfindung, da sie durch die Auswahl spezieller pharmazeutisch -technischer Zusatzmaßnahmen wie etwa den Einsatz spezieller Teilchenformen und Teilchengrößenverteilungen, besondere Preßbedingungen, Agglomerationsverfahren etc. ebenfalls zu einer erfindungsgemäßen Schichttablette führen können.

Der wesentliche Vorteil einer Zubereitung nach der Erfindung ist demnach darin zu sehen, daß eine damit hergestellte Schichttablette unbeschadet einer mechanischen Walk-Beanspruchung im Gastrointestinal-Trakt ihren Zusammenhalt bewahrt und somit eine gleichmäßigretardierende Freisetzung ihres Wirkstoffgehaltes bis zu dessen Erschöpfung beibehält.

## Patentansprüche

1. Orale Zubereitung, enthaltend in einer in wäßrigem Medium quellbaren Matrix wenigstens einen pharmazeutischen Wirkstoff, der aus der Matrix bei deren Quellung retardiert in das wässrige Medium freigesetzt wird, während die Matrix beim Freisetzungsprozess weitgehend zerfallsresistent bleibt, **dadurch gekennzeichnet, daß** sie eine Schichttablette mit aufeinander haftenden Schichten ist, von welchen wenigstens eine Schicht die quellbare Matrixschicht und wenigstens eine weitere Schicht eine Hilfs- und/oder Trägerschicht ist, und daß die quellbare Matrixschicht einen Anteil von quervernetzter Amylose enthält.

2. Zubereitung nach Anspruch 1, **dadurch gekennzeichnet, daß** der Anteil von quervernetzter Amylose in der Matrixschicht zwischen 20 und 99,5 Gew.-% beträgt.

3. Zubereitung nach Anspruch 1, **dadurch gekennzeichnet, daß** die Freisetzungsgeschwindigkeit über den größeren Teil der Freisetzungsdauer weitgehend konstant ist.

4. Zubereitung nach Anspruch 1, **dadurch gekennzeichnet, daß** sie mehr als einen Wirkstoff enthält.

5. Zubereitung nach einem oder mehreren der Ansprüche 1 bis 4, **dadurch gekennzeichnet, daß** die an die quellbare Matrixschicht angrenzende Schicht bzw. Schichten während der Wirkstofffreisetzung weitgehend zerfallsresistent ist/sind.

6. Zubereitung nach einem oder mehreren der Ansprüche 1 bis 5, **dadurch gekennzeichnet, daß** sie als Kern einer Film- oder Manteltablette ausgebildet ist.

## Claims

1. Oral preparation, comprising at least one pharmaceutical active compound in a matrix which is swellable in aqueous medium, which is released from the matrix into the aqueous medium in a delayed manner on swelling thereof, while the matrix remains largely resistant to disintegration during the release process, **characterized in that** it is a layered tablet with layers adhering to one another, of which at least one layer is the swellable matrix layer and at least another layer is an auxiliary and/or excipient layer, and **in that** the swellable matrix layer contains an amount of crosslinked amylose.

2. Preparation according to Claim 1, **characterized in that** the amount of crosslinked amylose in the matrix layer is between 20 and 99.5% by weight.

3. Preparation according to Claim 1, **characterized in that** the release rate over the greater part of the release period is largely constant.

4. Preparation according to Claim 1, charactertized in that it contains more than one active compound.

5. Preparation according to one or more of Claims 1 to 4, **characterized in that** the layer or layers 'adjacent to the swellable matrix layer is/are largely resistant to disintegration during the release of active compound.

6. Preparation according to one or more of Claims 1 to 5, **characterized in that** it is formed as a core of a film-coated or press-coated tablet.

## Revendications

1. Préparation orale renfermant, dans une matrice capable de gonflement dans un fluide aqueux, au moins une substance pharmaceutique qui est libérée de manière retardée dans le fluide aqueux par la matrice lors de son gonflement, la matrice restant largement résistante à la désagrégation au cours du processus de libération, **caractérisée en ce qu'**elle est un comprimé stratifié présentant des couches adhérant les unes aux autres dont au moins une couche est la couche de matrice apte au gonflement et au moins une autre couche est une couche accessoire et/ou de support, et **en ce que** la couche de matrice apte au gonflement contient une proportion d'amylose réticulée.

2. Préparation selon la revendication 1, **caractérisée en ce que** la teneur en amylose réticulée dans la couche de matrice est comprise entre 20 et 99,5 % en poids.

3. Préparation selon la revendication 1, **caractérisée en ce que** la vitesse de libération est largement constante sur la plus grande partie de la durée de la libération.

4. Préparation selon la revendication 1, **caractérisée en ce qu'**elle contient davantage qu'une substance active.

5. Préparation selon l'une ou plusieurs des revendications 1 à 4, **caractérisée en ce que** la ou les couches adjacentes à la couche de matrice apte au gonflement sont largement résistantes à la désagrégation pendant la libération de la substance active.

6. Préparation selon l'une ou plusieurs des revendications 1 à 5, **caractérisée en ce qu'**elle est configurée comme noyau d'un comprimé à film ou enveloppe.
